Europäisches Patentamt

European Patent Office

Office européen des brevets

(11) Numéro de publication: **0 299 847**
**A1**

(12) **DEMANDE DE BREVET EUROPEEN**

(21) Numéro de dépôt: 88401747.6

(22) Date de dépôt: 05.07.88

(51) Int. Cl.4: **C 12 N 1/00**
C 12 N 1/18

(30) Priorité: **17.07.87 FR 8710133**

(43) Date de publication de la demande:
**18.01.89 Bulletin 89/03**

(84) Etats contractants désignés:
**AT BE CH DE ES FR GB GR IT LI LU NL SE**

(71) Demandeur: **GROUPEMENT D'INTERET ECONOMIQUE
DIT: ETHANOL RECHERCHES ET DEVELOPPEMENTS
27-29, rue Chateaubriand
F-75383 Paris Cédex 08 (FR)**

(72) Inventeur: **De Baynast, Régis
35, rue de l'Ermitage
F-78 000 Versailles (FR)**

**Brouard, François
4, rue E.Patas d'Illiers
F-45 160 Olivet (FR)**

**Thierry, Anne
6, le Mont Lory - Chemin de Mont-Louis
F-69 230 Saint Genis Laval (FR)**

(74) Mandataire: **Bourgognon, Jean-Marie et al
Cabinet Flechner 22, Avenue de Friedland
F-75008 Paris (FR)**

(54) **Procédé de régénération et de décontamination de levures utilisées dans des installations de fermentation.**

(57) On les traite en milieu acide à un pH de 2 pendant moins de cinq minutes dans un réacteur piston (C) après les avoir concentrées dans un décanteur centrifuge(B).

## Description

**Procédé de régénération et de décontamination de levures utilisées dans des installations de fermentation.**

L'invention concerne les procédés de régénération et de décontamination de levures utilisées dans des installations de fermentation.

Un procédé connu de régénération et de décontamination des levures consiste à les mettre en contact avec un milieu acide, notamment une solution aqueuse sulfurique. Il se produit une désorption des macromolécules adhérant aux levures et une destruction des germes contaminants (U.S.A. 4008335).

Or, on s'est maintenant aperçu que ce traitement acide tue, certes, les bactéries et autres germes, mais est aussi nocif pour les levures.

L'invention pallie cet inconvénient par le procédé défini à la revendication 1.

On a trouvé qu'à ces conditions de pH et de durées de contact avec le milieu acide, les levures se conservent pour l'essentiel et qu'en tout cas les pertes sont bien moindres. Les microorganismes sont tués et les macro molécules désorbent dès que les durées de contact dépassent 0,5 minutes et, de préférence 1 minute.

On diminue d'autant plus la durée de contact que le pH est plus bas. On opère en général entre 15 et 40° C.

Les milieux acides sont généralement des solutions aqueuses concentrées d'acides minéraux, tels que l'acide phosphorique et l'acide sulfurique. La concentration en acide est ajustée en fonction du pH souhaité. Dans le cas d'une fermentation alcoolique, la concentration est de préférence telle que l'apport d'eau est inférieur à la quantité d'eau de dilution du sirop de sucres nécessaire à une fermentation produisant des vins titrant moins de 10 % d'éthanol. L'acide qui convient bien est l'acide sulfurique concentré.

Pour se rendre maître de la durée de contact, on ne saurait mélanger simplement les levures et le milieu acide sans autre précaution, puisqu'alors certaines levures se trouvant dans des zones mortes de mélange seraient en contact trop longtemps avec le milieu acide, alors que d'autres ne le seraient qu'insuffisamment, même si la durée moyenne de contact satisfait à la condition prévue par l'invention. L'invention prévoit d'effectuer la mise en contact en continu dans un réacteur en écoulement piston [plug flow (ou piston flow) reactor]. On rappelle qu'un mélange est en écoulement piston lorsqu'il progresse dans un réacteur par tranches parallèles et indépendantes n'échangeant pas de matière (diffusion axiale négligeable) à la manière d'un piston dans un cylindre. On trouvera une description de réacteurs à écoulement piston aux pages 56 et 57 de l'ouvrage de Jacques Villermaux intitulé "Génie de la réaction chimique conception et fonctionnement des réacteurs" Editions Lavoisier TEC & DOC.

On ne peut pas mettre en oeuvre un réacteur en écoulement piston susceptible de recevoir, à l'échelle industrielle, avec un débit convenable, la suspension diluée de levures, qui sort directement d'un fermenteur, par exemple de production d'éthanol. Le volume à traiter impliquerait un réacteur démesurément long. C'est pourquoi, dans un mode d'exécution perfectionné de l'invention, on procède, en amont du stade de mise en contact avec un milieu acide, à une concentration de la suspension des levures.

A cet effet, il est avantageux d'utiliser des levures floculantes. Ces levures ont la propriété de défloculer en milieu acide, leur pH de défloculation étant supérieur à celui auquel on procède à la mise en contact. Un pH de défloculation inférieur à 3, par exemple, convient bien. L'emploi de levures floculantes permet de bien concentrer les levures floculantes, de préférence dans un décanteur centrifuge horizontal. Avantageusement, le décanteur centrifuge tourne à des vitesses donnant des accélérations de 2000 à 3000 g et notamment 2400 à 2600 g. Il fournit une "boue" de levures ayant une teneur en levures supérieure à 150 g/l et, de préférence, supérieure à 200 g/l, par exemple comprise entre 200 et 250 g/l. Lorsque cette boue de levures floculées et concentrées passe ensuite dans le réacteur de mise en contact avec un milieu acide, elles défloculent, sous l'effet même de ce milieu, la combinaison de l'acidité et de l'emploi de levures floculantes ayant ainsi pour fonction à la fois de régénérer et de décontaminer les levures et de rendre l'opération de brève mise en contact faisable industriellement, par la grande concentration préalable que la floculation rend possible et par l'absence de décantation pendant le passage dans le réacteur en écoulement piston.

Les levures floculantes sont de préférence du genre Saccharomyces, telles que Saccharomyces cerevisiae., Schizosaccharomyces pombe et Saccharomyces uvarum.

L'invention vise également une installation · de fermentation comprenant un fermenteur ayant une entrée et une sortie et un circuit de régénération relié à l'entrée et à la sortie, caractérisée en ce que le circuit comprend successivement, de la sortie vers l'entrée, un dispositif de concentration des levures et un réacteur en écoulement piston.

L'invention s'applique particulièrement aux installations de production d'éthanol par fermentation.

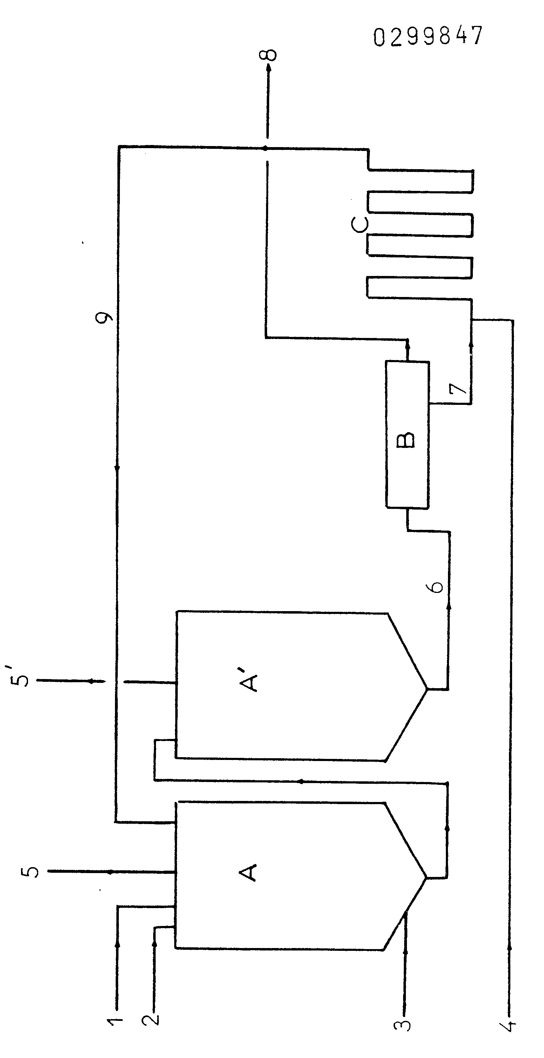

La figure unique du dessin annexé est un schéma d'une installation suivant l'invention.

La fermentation est mise en oeuvre dans une ou plusieurs cuves en cascade A, A',.... .

De façon continue, on alimente la cuve A en sirop de sucre concentré par un conduit 1 et en une fraction de l'eau et/ou des vinasses nécessaires à l'obtention d'un jus alcoolisé au titre souhaité par un conduit 2. De l'air peut être injecté en 3. Le gaz carbonique produit au cours de la fermentation est évacuée en 5, 5',... .

De façon continue, on extrait par une sortie un jus alcoolisé, appelé "vin" 6. Il contient des levures floculantes dont la concentration est la même que celle établie dans la cuve A. Le jus est envoyé dans un décanteur horizontal centrifuge B. L'accélération centrifuge est inférieure à 3000 g. La fraction clarifiée ou délevurée du· vin est extraite en 8 pour être envoyée en colonne à distiller. Le débit de vin dans le conduit 8 est égal à la somme des débits de liquide entrant par les conduit 1, 2 et 4. Le vin clarifié a une teneur en levures inférieure à $10^7$ levures par ml. La fraction des levures concentrées est extraite en 7. La concentration en levures est supérieure à 25 % (p/p). Le concentré est dilué avec une eau acidifiée 4 de façon que le pH se situe entre 1 et 3. Les levures défloculent. En conséquence de quoi, un traitement acide dans un réacteur piston de type lyre C peut être effectué. La vitesse du fluide dans le réacteur peut être lente, sans que les levures ne décantent. Le temps de séjour hydraulique dans le réacteur est généralement compris entre 1 et 5 minutes, mais peut atteindre 60 minutes lorsque le pH est compris entre 2 et 3. Les caractéristiques de concentration initiale en levures et de défloculation en milieu acide permettent de contrôler parfaitement la durée du traitement. Le flux de levures acidifiées retourne au réacteur A par le conduit d'entrée 9. Dans A, le pH du milieu réactionnel est compris entre 4 et 5 du fait de la dilution du contenu du conduit 9 par les apports des conduits 1 et 2, et surtout du pouvoir tampon des substances contenues dans le sirop arrivant par le conduit 1. Cette augmentation de pH conduit à une refloculation instantanée des levures apportées par 9. Les propriétés de décantation des levures qui ont permis d'obtenir leur exceptionnel taux de concentration dans le décanteur centrifuge horizontal sont donc retrouvées.

Le traitement acide des levures dans le réacteur piston permet un contrôle exact de la destruction des germes contaminants, du lavage des levures, ainsi que de leur mortalité. Il présente l'avantage de pouvoir être mis en oeuvre de façon continue sans affecter la productivité globale de l'installation de fermentation. Il répond donc aux exigences des nouvelles unités de production d'éthanol agricole à forte productivité, devant fonctionner pendant de longues périodes.

L'exemple suivant illustre l'invention.

### Exemple

#### Fermentation de mélasse

#### Conditions de fermentation

\* L'essai a été effectué sur une unité pilote de fermentation continue en une cuve de type CSTR (continuous stirred tank reactor) d'un volume utile de 5000 litres.

Le réacteur est équipé de systèmes de régulation :

- du pH, par addition d'acide sulfurique concentré,
- de température,
- de niveau et de débit.

Les débits d'alimentation en eau et en substrat sont régulés en continu.

Une rampe d'aération permet l'aération du milieu de fermentation.

\* Les conditions de fermentation sont les suivantes :
- pH : 4,8 ± 0,5
- température : 30° C ± 1° C
- aération : 1 vvh (volume d'air par volume de milieu et par heure).

Le substrat fermenté est de la mélasse de betteraves à 54 % de sucres fermentescibles totaux (exprimés en unités-glucose).

L'alimentation du réacteur a été régulée à 330 l/h, avec un moût à 137 g de saccharose/l (soit 144g/l de sucres fermentescibles totaux).

Aucune addition de sels (sulfate de magnésie, phosphate d'ammonium,...) n'a été effectuée.

Le taux de conversion du sucre, pour un temps de séjour hydraulique de 15 heures, a été de 99 %.

Le titre alcoolique du vin est de 8,5° GL (6,63 g/l), ce qui correspond à un rendement de transformation du sucre en éthanol de 62 litres d'éthanol pour 100 g de saccharose (ce rendement est égal à 92 % du rendement théorique, dit "de Gay-Lussac").

#### Mise en oeuvre du procédé de régénération/décontamination des levures

La souche de levure utilisée est une souche Saccharomyces cerevisiae. Sa concentration dans la cuve a été stabilisée à 20 g de matière sèche/l, c'est-à-dire $5.10^8$ cellules/ml de milieu.

Le milieu de fermentation est envoyé dans un décanteur centrifuge horizontal fourni par Guinard de marque D1L à un débit de 700 1/h.

La vitesse de rotation du bol du décanteur a été fixée à 5000 t/mn, ce qui crée une accélération centrifuge de 2500 g. La vitesse relative de rotation de la vis convoyeuse par rapport au bol peut être réglée à 10, 20 ou 30 t/mn. Elle a été fixée à 30 t/mn afin d'augmenter la vitesse d'évacuation des boues.

La couronne de réglage déterminant le diamètre de débordement du liquide clair a été montée avec un diamètre faible (110 mm), afin de clarifier au maximum l'effluent et de maximiser ainsi le taux de recyclage des levures.

Le vin clarifié issu du séparateur, au débit de 650 l/h, ne contient plus que 4 à $7.10^6$ levures/ml.

La "boue" de levures concentrées contient 300 à 320 g/l de matière séche totale, dont 250 g/l de levure. La viscosité des boues est de l'ordre de 30 Pa.s. (à 20° C). Les levures concentrées sont réintroduites en totalité dans le réacteur de fermentation. Le débit de "boue" des levures est égal à 50 l/h.

Le séparateur centrifuge utilisé permet donc d'obtenir un rendement de séparation (le rapport de la masse des insolubles des sédiments en sortie à la masse des insolubles en alimentation) v-isin de 99 % et d'atteindre un facteur de concentration (rapport de la concentration en insolubles des sédiments en sortie à la concentration des insolubles en alimentation) des levures égal à 12.

Le taux de contamination bactérien du milieu de culture s'est élevé à $2.10^6$ germes/ml en 15 jours (les contaminants ont été dénombrés sur gélose MRS, adapté au dénombrement de la flore de type lactique qui se développe en priorité à pH bas).

Le système de mise en contact avec un milieu acide consiste en un réacteur piston permettant un temps de séjour hydraulique des levures de 5 mn à pH = 1,8.

Le concentré de levures est additionné d'un volume égal d'eau acidifiée, soit 50 l permettant d'obtenir une crème de levures de viscosité voisine de 0,3 Pa.s. au pH désiré.

Les quantité d'acide ($H_2SO_4$, 36 N) nécessaires au traitement sont de 11 g/l de crème diluée au 1/2.

On constate au cours du traitement :
- une défloculation totale des agrégats de boues,
- une réduction significative du nombre de germes.

La "boue" de levure acidifiée et diluée est réintroduite dans la cuve de fermentation après traitement.

Après réintroduction de la "boue" de levure dans la cuve de fermentation, le phénomène de floculation se reproduit de façon instantanée.

Le traitement proposé permet de conserver un excellent rendement de fermentation alcoolique sur de longues périodes de fermentation, d'annuler presque totalement les risques d'infection, malgré un système de fermentation non stérile, de réduire le taux de contaminants de façon significative et de ne perdre que très peu de levures.

## Revendications

1. Procédé de régénération et de décontamination de levures en les mettant en contact avec un milieu acide, caractérisé en ce qu'il consiste à ne mettre les levures en contact avec le milieu acide, à un pH compris entre 1,2 et 3, que pendant une durée inférieure à 30 minutes et alors qu'elles sont en écoulement dans un réacteur piston.

2. Procédé suivant la revendication 1, caractérisé en ce qu'il consiste à ne mettre les levures en contact avec le milieu acide, à un pH compris entre 1,2 et 1,7, que pendant une durée inférieure à 5 minutes.

3. Procédé suivant la revendication 1 ou 2, caractérisé en ce qu'il consiste à mettre les levures en contact avec le milieu acide pendant au moins une demi-minute et, de préférence, pendant au moins une minute.

4. Procédé suivant l'une des revendications 1 à 3, caractérisé en ce qu'il consiste à concentrer une suspension de levures floculantes à mettre en contact avec le milieu acide en une boue ayant une concentration en levures d'au moins 150 g/l et, de préférence, d'au moins 200g/l, avant de la mettre en contact avec le milieu acide.

5. Procédé suivant la revendication 4, caractérisé en ce qu'il consiste à effectuer la concentration dans un décanteur centrifuge horizontal.

6. Procédé suivant la revendication 4 ou 5, caractérisé en ce que les levures floculantes sont du genre saccharomyces.

7. Installation de fermentation comprenant un fermenteur ayant une entrée et une sortie et un circuit de régénération relié à l'entrée et à la sortie, caractérisé en ce que le circuit comprend successivement, de la sortie vers l'entrée, un dispositif de concentration des levures et un réacteur en écoulement piston.

8. Installation suivant la revendication 7, caractérisé en ce que le dispositif de concentration est un décanteur centrifuge.

0299847

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | US-A-4 008 335  (E. AKERMAN et al.)<br>* Revendications 1,4; colonne 1, ligne 64 - colonne 2, ligne 4; colonne 4, lignes 8-23; exemple 1 *<br>--- | 1-4,6,7 | C 12 N    1/00<br>C 12 N    1/18 |
| Y | CHEMICAL ABSTRACTS, vol. 92, no. 13, 31 mars 1980, page 567, résumé no. 109083e, Columbus, Ohio, US; M. MORENO et al.: "Alcohol fermentation in strict anaerobiosis in a plug flow fermentor: effect of cell recycling", & BIOTECHNOL. LETT. 1979, 1(12), 483-8<br>* En entier *<br>--- | 1-4,6,7 | |
| A | CHEMICAL ABSTRACTS, vol. 84, no. 1, 5 janvier 1976, page 298, résumé no. 3268p, Columbus, Ohio, US; P.C. BONNIN: "Effects of brewer's yeast purified with sulfuric acid on fermentative power", & TEC. LAB. 1975, 40, 166-75<br>* En entier *<br>--- | 1,6 | |
| A | GB-A-1 234 317  (WESTFALIA)<br>* Revendications 1,5; figures *<br>--- | 5,8 | **DOMAINES TECHNIQUES RECHERCHES (Int. Cl.4)**<br><br>C 12 N |
| A | GB-A-1 246 231  (STANDARD BRANDS)<br>----- | | |

Le présent rapport a été établi pour toutes les revendications

| Lieu de la recherche | Date d'achèvement de la recherche | Examinateur |
|---|---|---|
| LA HAYE | 26-10-1988 | WIESER, M.R.J. |